# EUROPEAN PATENT APPLICATION

(11) **EP 3 855 139 A1**
(43) Date of publication of application: **28.07.2021**
(21) Application number: 18933744.7
(22) Date of filing: 21.09.2018
(51) Int. Cl.: G01J 1/44, G01J 1/42, G01N 21/3504

(54) **SIGNAL PROCESSING CIRCUIT, MEASUREMENT DEVICE, AND SIGNAL PROCESSING METHOD**

(71) Applicant: Otsuka Pharmaceutical Co., Ltd., Tokyo 101-8535 (JP)
(72) Inventor: KAJIWARA, Shinpei, Hirakata-shi, Osaka 573-1132 (JP); FUJIMOTO, Takashi, Hirakata-shi, Osaka 573-1132 (JP); IKEDA, Atsushi, Hirakata-shi, Osaka 573-1132 (JP)
(74) Representative: Hoffmann Eitle
(86) International application number: PCT/JP2018/034959
(87) International publication number: WO 2020/059102

(57) **Abstract**

A signal processing circuit (40) processes a signal based on a quantity of light detected by an infrared detector. The signal processing circuit (40) includes an IV conversion circuit (41), a filter circuit (43), and a differential converter circuit (44). The IV conversion circuit (41) converts a signal of a current value provided from the infrared detector to a signal of a voltage value. The filter circuit (43) allows passage of a signal containing a predetermined frequency component among signals converted by the IV conversion circuit (41). The differential converter circuit (44) generates a reverse-phase signal of a passed signal that has passed through the filter circuit (43) and provides a differential signal including the passed signal and the reverse-phase signal.

## Description

### TECHNICAL FIELD

The present invention relates to a signal processing circuit, a measurement apparatus, and a signal processing method.

### BACKGROUND ART

A measurement apparatus that uses an infrared detector for diagnosis of a disease has recently been developed in the medical field. For example, a measurement apparatus that measures variation in concentration ratio between isotopes with an infrared detector after administration of a drug containing the isotopes to a living body, finds a metabolic rate of the living body, and uses the metabolic rate for diagnosis of a disease has recently been developed.

In order to detect a quantity of infrared light from a light source with an infrared detector and to use a result of detection for diagnosis of a disease in such a measurement apparatus, a signal provided from the infrared detector should have an S/N ratio to some extent. In order to improve the S/N ratio of the signal provided from the infrared detector, the quantity of light detected by the infrared detector may be increased. In order to increase the quantity of light detected by the infrared detector, however, an amount of a sample measured by the measurement apparatus should be increased, which imposes burden on a subject.

The S/N ratio of the signal may be improved by processing a signal provided from the infrared detector with a signal processing circuit. For example, PTL 1 (Japanese Patent Laying-Open No. 4-357423) or PTL 2 (Japanese Patent Laying-Open No. 2016-5067) discloses as the signal processing circuit, a signal processing circuit including an amplifier circuit that amplifies a signal provided from a detector.

### CITATION LIST

### PATENT LITERATURE

PTL 1: Japanese Patent Laying-Open No. 4-357423
PTL 2: Japanese Patent Laying-Open No. 2016-5067

### SUMMARY OF INVENTION

### TECHNICAL PROBLEM

In the signal processing circuit disclosed in PTLs 1 and 2, however, the amplifier circuit amplifies a signal provided from the detector with respect to a reference voltage. Therefore, when an error is produced in the reference voltage, the error may directly affect the signal provided from the detector. In addition, in the signal processing circuit disclosed in PTLs 1 and 2, a difference from the reference voltage defines a dynamic range of the signal provided from the detector, and hence a signal wide in dynamic range has not been obtained.

An object of the present invention is to provide a signal processing circuit, a measurement apparatus, and a signal processing method that can achieve improvement in S/N ratio of a signal obtained by a detector and can obtain a signal wider in dynamic range.

### SOLUTION TO PROBLEM

According to one aspect of the present invention, a signal processing circuit that processes a signal based on a quantity of light detected by an infrared detector includes a current-voltage conversion circuit that converts a signal of a current value provided from the infrared detector to a signal of a voltage value, a filter circuit that allows passage of a signal containing a predetermined frequency component among signals converted by the current-voltage conversion circuit, and a differential circuit that generates a reverse-phase signal of a passed signal that has passed through the filter circuit and provides a differential signal including the passed signal and the reverse-phase signal.

Preferably, an analog-digital conversion circuit that converts the differential signal provided from the differential circuit to a digital signal is further included.

Preferably, the analog-digital conversion circuit sets a resolution at which the differential signal is converted to the digital signal to be higher than a resolution at which the passed signal is converted to the digital signal.

Preferably, an amplifier circuit that amplifies a signal converted by the current-voltage conversion circuit is further included and the signal amplified by the amplifier circuit is provided to the filter circuit.

Preferably, an amplifier circuit that amplifies a signal is further included between the filter circuit and the differential circuit.

Preferably, the signal based on the quantity of light detected by the infrared detector is a signal obtained by detection by the infrared detector, of a quantity of infrared light from a light source intensity of which is sinusoidally modulated by a light modulator.

Preferably, the infrared detector is a photovoltaic element.

According to another aspect of the present invention, a measurement apparatus that measures a component gas concentration ratio of gas to be measured that contains two types of component gas in isotopic relation with each other includes a cell in which the gas to be measured is stored, a light source that emits infrared light to be transmitted through the cell, a light modulator that sinusoidally modulates intensity of infrared light from the light source, an optical filter that allows transmission of a wavelength suitable for each type of component gas, of infrared light transmitted through the cell, an infrared detector that detects a quantity of light transmitted through the optical filter, the above-described signal processing circuit that processes a signal based on the quantity of light detected by the infrared detector, and a computing circuit that finds absorbance of the wavelength suitable for component gas based on the signal processed by the signal processing circuit and calculates the component gas concentration ratio.

Preferably, the two types of component gas contained in the gas to be measured are carbon dioxide ¹³CO₂ and carbon dioxide ¹²CO₂.

According to yet another aspect of the present invention, a signal processing method of processing a signal provided from an infrared detector includes converting a signal detected as a current value by the infrared detector to a signal of a voltage value, allowing passage of a signal containing a predetermined frequency component among signals converted to voltage values, and generating a reverse-phase signal of a signal that has passed through and providing a differential signal including the signal that has passed through and the reverse-phase signal to an analog-digital conversion circuit.

### ADVANTAGEOUS EFFECTS OF INVENTION

With the signal processing circuit according to the present technology, with a differential circuit that provides a differential signal including a passed signal and a reverse-phase signal, the S/N ratio of a signal obtained by the detector can be improved and a signal wider in dynamic range can be obtained.

### BRIEF DESCRIPTION OF DRAWINGS

Fig. 1 is a schematic diagram for illustrating a configuration of a measurement apparatus according to the present embodiment.
Fig. 2 is a schematic diagram for illustrating a configuration of a signal processing circuit according to the present embodiment.
Fig. 3 is a block diagram for illustrating a configuration of a computing circuit according to the present embodiment.
Fig. 4 is a diagram for illustrating signal processing in the signal processing circuit according to the present embodiment.
Fig. 5 is a flowchart for illustrating a signal processing method in the signal processing circuit according to the present embodiment.
Fig. 6 shows a graph for illustrating an amount of variation of a signal processed by the signal processing circuit according to the present embodiment.
Fig. 7 is a schematic diagram for illustrating a configuration of a signal processing circuit according to a modification.

### DESCRIPTION OF EMBODIMENTS

The present embodiment will be described in detail below with reference to the drawings. The same or corresponding elements in the drawings have the same reference characters allotted.

### (Application)

An apparatus that measures variation in concentration ratio between isotopes based on light absorption characteristics of the isotopes after administration of a drug containing the isotopes to a living body, finds a metabolic rate of the living body, and uses the metabolic rate for diagnosis of a disease will be described by way of example of a measurement apparatus according to the present embodiment. Specifically, a measurement apparatus used for diagnosing whether or not Helicobacter pylori (HP) alleged as a cause of gastric ulcer or gastritis exists in the stomach of a subject will be described.

Various methods have been proposed as methods of diagnosing whether or not HP exists in a subject. The measurement apparatus according to the present embodiment uses a property of HP to decompose urea into carbon dioxide and ammonia with its high urease activities to diagnose existence of HP based on variation in concentration ratio of ¹³CO₂ resulting from decomposition of urea marked with an isotope ¹³C administered to a subject.

In addition to a carbon isotope having a mass number of 12, there are carbon isotopes having mass numbers of 13 and 14,. Among these isotopes, the isotope ¹³C having the mass number of 13 is not radioactive, exists in a stable manner, and is easy to handle. Therefore, urea administered to the subject is marked with the isotope ¹³C. When HP exists in the stomach of the subject, HP decomposes the isotope into ¹³CO₂ and ammonia. ¹³CO₂ resulting from decomposition is emitted as being contained in the breath of the subject. Therefore, by measuring the concentration ratio of ¹³CO₂ contained in the breath of the subject, whether or not HP exists in the stomach of the subject can be diagnosed.

The concentration ratio between ¹³CO₂ and ¹²CO₂ contained in air is 1:100. Therefore, the measurement apparatus according to the present embodiment is required to accurately measure the concentration ratio between ¹³CO₂ and ¹²CO₂. The measurement apparatus according to the present embodiment uses infrared spectrophotometry as the method of finding a concentration ratio between ¹³CO₂ and ¹²CO₂ and includes two long and short cells in which absorption by ¹³CO₂ in one cell is equal to absorption by ¹²CO₂ in the other cell. The measurement apparatus measures a quantity of transmitted light (transmitted light quantity) by irradiating each cell with infrared light at a wavelength suitable for analysis and finding variation in concentration ratio in the breath of a subject with respect to the concentration ratio in air. Japanese Patent Publications Nos. 61-42219 and 61-42220 disclose a method of measuring a concentration ratio between ¹³CO₂ and ¹²CO₂.

In order to accurately measure the concentration ratio between ¹³CO₂ and ¹²CO₂, the measurement apparatus according to the present embodiment is provided with a signal processing circuit that processes a signal based on a quantity of light transmitted through the cell and detected by the detector to improve the S/N ratio of the signal and to obtain a signal wider in dynamic range.

An example in which a urea diagnostic drug marked with the isotope ¹³C is administered to a subject and thereafter the concentration ratio of ¹³CO₂ in the breath is measured by spectrophotometry will be described in detail below with reference to the drawings. Initially, the breath of the subject before administration of the urea diagnostic drug is collected in a breath bag as base gas. Thereafter, the urea diagnostic drug is orally administered to the subject, and after twenty minutes, the breath of the subject is collected in a breath bag as sample gas.

The breath bag for sample gas and the breath bag for base gas are each set onto a prescribed nozzle of the measurement apparatus and the concentration ratio between ¹³CO₂ and ¹²CO₂ is measured. Base gas and sample gas represent gas to be measured by the measurement apparatus.

### (Configuration of Measurement Apparatus)

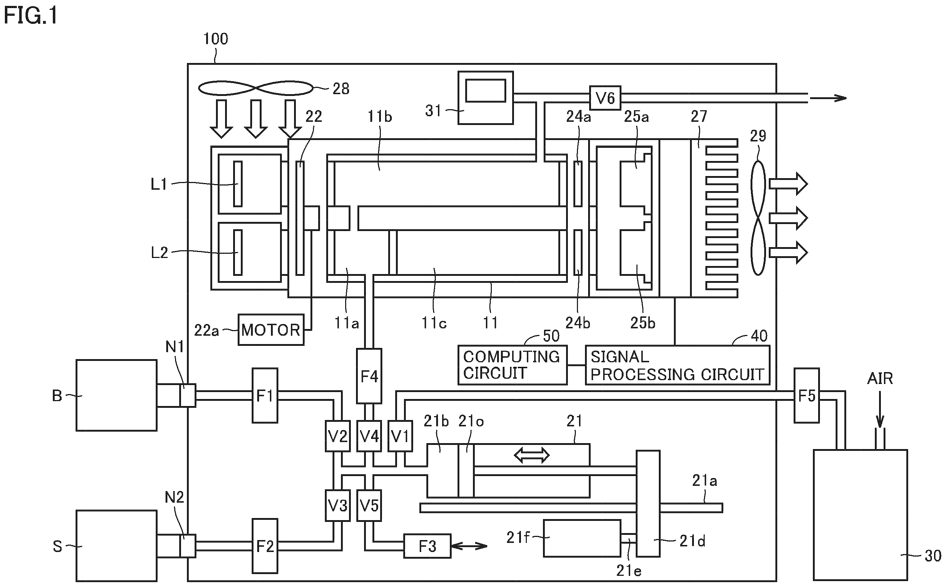

Fig. 1 is a schematic diagram for illustrating a configuration of a measurement apparatus 100 according to the present embodiment. In measurement apparatus 100, a breath bag B for base gas and a breath bag S for sample gas are set onto nozzles N1 and N2, respectively. Nozzle N1 is connected through a pipe (for example, a metal pipe) to a filter F1 and a valve (for example, an electromagnetic valve) V2. Filter F1 is a filter for removing foreign matters other than base gas contained in breath bag B. Nozzle N2 is connected through a pipe to a filter F2 and a valve V3. Valve V2 and valve V3 are connected to a gas injector 21 through a single pipe. Filter F2 is a filter for removing foreign matters other than base gas contained in breath bag S.

Valves VI, V4, and V5 are connected to pipes connected to gas injector 21. Valve V1 is connected to a filter F5 and a reference gas supply portion 30 through a pipe. Reference gas supply portion 30 includes a carbonic acid gas absorption portion that uses, for example, soda lime (a mixture of sodium hydroxide and calcium hydroxide) as a carbonic acid gas absorbing agent. Therefore, reference gas supply portion 30 can supply reference gas that has absorbed carbon dioxide from air taken in from the outside to gas injector 21. Filter F5 is a dust filter that removes foreign matters from reference gas supplied to gas injector 21.

Valve V4 is connected to a filter F4 and a cell 11 through a pipe. Valve V5 is connected to a filter F3 through a pipe. Filter F3 is provided at an air inlet and removes foreign matters from taken air. Filter F4 is a dry filter that removes moisture from reference gas, second gas to be measured, and second gas to be measured that are supplied to gas injector 21.

Gas injector 21 is in such a structure that a cylinder 21b containing a piston 21c is arranged on a base 21a and a movable nut 21d coupled to piston 21c, a feed screw 21e meshed with nut 21d, and a pulse motor 2 1f that rotates feed screw 21e are provided under base 21a.

Pulse motor 21f is driven to rotate forward or reverse by a not-shown drive circuit. As feed screw 21e is rotated by rotation of pulse motor 21f, nut 21d moves forward or rearward depending on a rotation direction to move piston 21c forward or rearward to any position. Therefore, introduction of gas to be measured into cylinder 21b and emission of gas to be measured from cylinder 21b can thus freely be controlled.

The other side of valve V4 is connected to a first sample cell 11a for measuring absorption by ¹²CO₂.First sample cell 11a is a short cell for measuring absorption by ¹²CO₂ in one cell of cell 11. Cell 11 additionally includes a long second sample cell 11b for measuring absorption by ¹³CO₂ and an auxiliary cell 11c. First sample cell 11a and second sample cell 11b communicate with each other. Gas guided to first sample cell 11a enters second sample cell 11b as it is and exhausted through a valve V6. Auxiliary cell 11c is filled with reference gas that absorbs no infrared rays and hermetically sealed. Instead of filling auxiliary cell 11c with reference gas and hermetically sealing the auxiliary cell, reference gas may be guided from reference gas supply portion 30 to the auxiliary cell and may constantly be fed at a constant flow velocity.

First sample cell 11a has a volume of approximately 0.1 ml and second sample cell 11b has a volume of approximately 3.7 ml. An end surface of cell 11 is provided with a sapphire transmission window through which infrared rays are transmitted. Cell 11 is connected to a pressure gauge 31 through a pipe. Pressure gauge 31 can measure a pressure of gas introduced into cell 11 by gas injector 21.

Light source apparatuses L1 and L2 that emit infrared light are provided on one end surface side of cell 11. Light source apparatuses L1 and L2 are each provided with two waveguides (not shown) for emitting infrared rays. Any method of generation of infrared light by light source apparatuses L1 and L2 may be applicable, and for example, a ceramic heater (a surface temperature of 450°C) can be employed. An optical chopper 22 that cuts off and allows passage of infrared light in constant cycles is provided between light source apparatuses L1 and L2 and cell 11. Optical chopper 22 can allow emission of infrared light to cell 11 in constant cycles (for example, 600 Hz) by being rotated by a motor 22a. In other words, optical chopper 22 is a light modulator that sinusoidally modulates intensity of infrared light emitted from light source apparatuses L1 and L2.

Infrared light emitted from light source apparatus L1 passes through second sample cell 1 1b and a quantity of light thereof is detected by an infrared detector 25a. A wavelength filter 24a is provided between second sample cell 11b and infrared detector 25a. Infrared light emitted from light source apparatus L2 passes through first sample cell 11a and auxiliary cell 11c and a quantity of light thereof is detected by an infrared detector 25b. A wavelength filter 24b is provided between first sample cell 11a and auxiliary cell 11c, and infrared detector 25b.

Wavelength filter 24a is designed to allow passage of infrared light at a wavelength of approximately 4412 nm for measuring absorption by ¹³CO₂ and wavelength filter 24b is designed to allow passage of infrared rays at a wavelength of approximately 4280 nm for measuring absorption by ¹²CO₂.Infrared detectors 25a and 25b are elements that detect a quantity of infrared light and they are photovoltaic elements. The photovoltaic element is an element that uses a phenomenon of generation of electromotive force as a result of irradiation of a substance with light (that is, a photovoltaic effect), and it is, for example, an InAsSb element that generates electromotive force upon receiving infrared light. In particular, the InAsSb element could realize fast response and high reliability because PN junction is adopted therein. Infrared detectors 25a and 25b may each be a photoconductive element an electrical resistance of which is varied by infrared light, such as a PbSe element.

The entire infrared detectors 25a and 25b are maintained at a constant temperature by a heater and a Peltier element 27. Fans 28 and 29 that ventilate measurement apparatus 100 are provided. Measurement apparatus 100 includes a signal processing circuit 40 that processes signals provided from infrared detectors 25a and 25b.

### (Configuration of Signal Processing Circuit)

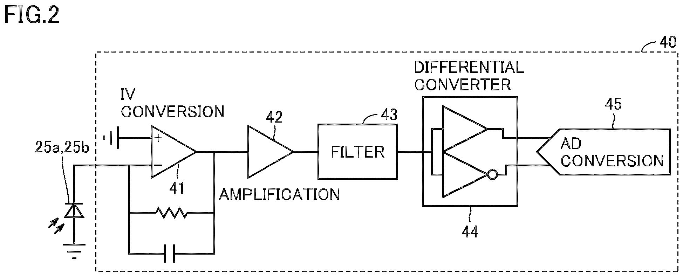

Fig. 2 is a schematic diagram for illustrating a configuration of signal processing circuit 40 according to the present embodiment. Signal processing circuit 40 includes a current-voltage conversion circuit (an IV conversion circuit) 41, an amplifier circuit 42, a filter circuit 43, a differential converter circuit (differential circuit) 44, and an analog-digital conversion circuit (an AD conversion circuit) 45.

IV conversion circuit 41 converts a signal of a current value provided from infrared detector 25a or 25b into a signal of a voltage value. Infrared detector 25a or 25b provides to IV conversion circuit 41, a signal of a current value in accordance with a quantity of transmitted light transmitted through cell 11. IV conversion circuit 41 provides a transmitted light quantity detected by infrared detector 25a or 25b as a signal of a voltage value.

Amplifier circuit 42 amplifies the signal converted by IV conversion circuit 41 to a signal that filter circuit 43 or differential converter circuit 44 can process. For example, when a signal converted by IV conversion circuit 41 is a signal having a maximum amplitude from 0 V to 2.5 V, amplifier circuit 42 amplifies a voltage value thereof by approximately two times to provide a signal having a maximum amplitude from 0 V to 5 V.

Since intensity of infrared light emitted from light source apparatuses L1 and L2 is sinusoidally modulated by optical chopper 22, the transmitted light quantity detected by infrared detectors 25a and 25b is sinusoidal. For example, when optical chopper 22 cuts off light from the light source at 600 Hz, the transmitted light quantity detected by infrared detectors 25a and 25b exhibits a sinusoidal wave at 600 Hz. Therefore, a signal provided from infrared detector 25a or 25b also exhibits a sinusoidal wave at 600 Hz, and a signal amplified by amplifier circuit 42 exhibits a sinusoidal wave having a maximum amplitude from 0 V to 5 V and a frequency of 600 Hz. Filter circuit 43 is a band pass filter that allows passage of a signal containing a frequency component (for example, 600 Hz) modulated by optical chopper 22. In other words, the signal containing a predetermined frequency component allowed to pass through filter circuit 43 is a signal containing the frequency component (for example, 600 Hz) modulated by optical chopper 22.

Differential converter circuit 44 generates a signal reverse in phase (a reverse-phase signal) to a signal (a passed signal) that has passed through filter circuit 43, and provides a differential signal including both of these signals (the passed signal and the reverse-phase signal) to AD conversion circuit 45. AD conversion circuit 45 converts a difference of the passed signal from the reverse-phase signal to a digital signal as a measurement result. Detailed processing in signal processing circuit 40 will be described later.

Referring back to Fig. 1, measurement apparatus 100 includes a computing circuit 50 that diagnoses whether or not HP exists based on a signal processed by signal processing circuit 40. In addition to diagnosing whether or not HP exists, computing circuit 50 controls measurement apparatus 100; it controls opening and closing of valves V1 to V6 and controls the circuit for driving pulse motor 21f. Computing circuit 50 controls representation on a not-shown display (for example, an LDC).

### (Configuration of Computing Circuit)

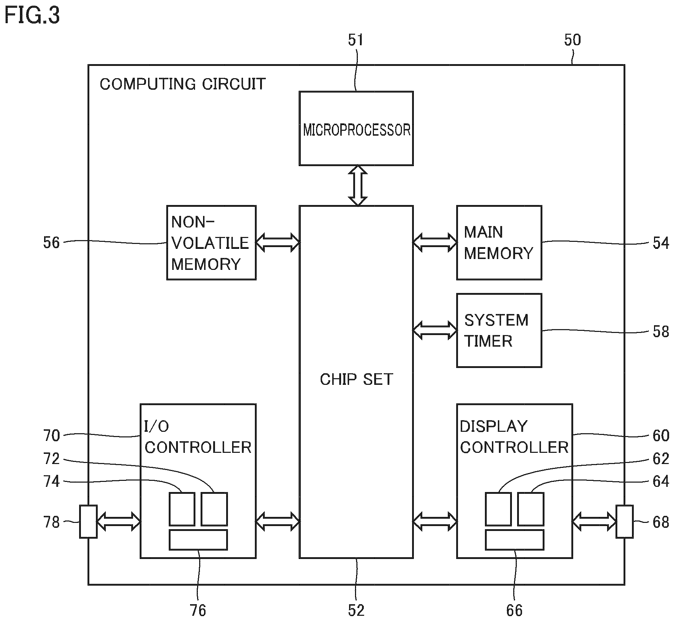

Fig. 3 is a block diagram for illustrating a configuration of computing circuit 50 according to the present embodiment. Referring to Fig. 3, computing circuit 50 includes a microprocessor 51, a chip set 52, a main memory 54, a non-volatile memory 56, a system timer 58, a display controller 60, and an I/O controller 70. Chip set 52 is coupled to other components through various buses.

Microprocessor 51 and chip set 52 are typically configured in conformity with a general-purpose computer architecture. Specifically, microprocessor 51 interprets and executes instruction codes sequentially supplied from chip set 52 in response to an internal clock. Chip set 52 exchanges internal data with various connected components and generates an instruction code necessary for microprocessor 51. Chip set 52 performs a function to cache data obtained as a result of computing processing in microprocessor 51.

Computing circuit 50 includes main memory 54 and non-volatile memory 56 as storage means.

Main memory 54 is a volatile storage area (a RAM) and holds various programs to be executed by microprocessor 51 after power of computing circuit 50 is turned on. Main memory 54 is used also as a work memory when microprocessor 51 executes various programs. Such a device as a dynamic random access memory (DRAM) or a static random access memory (SRAM) is employed as main memory 54.

Non-volatile memory 56 holds a real-time operating system (OS), a system program for measurement apparatus 100, a user program, an operation program, and data such as a setting parameter in a non-volatile manner. These programs and data are copied to main memory 54 so as to be accessible from microprocessor 51 as necessary. A semiconductor memory such as a flash memory can be employed as non-volatile memory 56. Alternatively, a magnetic recording medium such as a hard disk drive or an optical recording medium such as a digital versatile disk random access memory (DVD-RAM) can also be employed.

System timer 58 generates an interrupt signal every constant cycle and provides the interrupt signal to microprocessor 51. Typically, depending on specifications of hardware, the system timer generates interrupt signals in a plurality of different cycles, however, it can also be set to generate interrupt signals in any cycles by means of an operating system (OS) or a basic input output system (BIOS).

Display controller 60 is connected to a display provided in measurement apparatus 100 with a connection portion 68 being interposed and controls the display. Display controller 60 includes a memory control circuit 62, a display control circuit 64, and a buffer memory 66.

Buffer memory 66 functions as a transmission buffer for transmission of display data provided to the display through display controller 60 and a reception buffer for reception of input data provided from the display (for example, a touch panel).

Memory control circuit 62 has output data transferred from main memory 54 to buffer memory 66 and has input data transferred from buffer memory 66 to main memory 54.

Display control circuit 64 performs processing for transmitting display data in buffer memory 66 to the connected display and performs processing for receiving input data and storing the input data in buffer memory 66.

I/O controller 70 is connected to a device that controls valves V1 to V6 or pulse motor 21f provided in measurement apparatus 100 and signal processing circuit 40 with a connection portion 78 being interposed, and controls output of a control signal to valves V1 to V6 or pulse motor 21f and input of a digital signal from signal processing circuit 40. I/O controller 70 includes a memory control circuit 72, a signal processing circuit 74, and a buffer memory 76.

Buffer memory 76 functions as a transmission buffer for transmission of a control signal provided to valves V1 to V6 or pulse motor 21f through I/O controller 70 and a reception buffer for reception of a digital signal provided from signal processing circuit 40.

Memory control circuit 72 has a control signal transferred from main memory 54 to buffer memory 76 and has a digital signal transferred from buffer memory 76 to main memory 54.

Signal processing circuit 74 performs processing for transmitting a control signal for buffer memory 76 to a device that controls valves V1 to V6 or pulse motor 21f connected to I/O controller 70 and signal processing circuit 40 and processing for receiving a digital signal and storing the digital signal in buffer memory 76.

### (Measurement Processing)

Measurement processing in measurement apparatus 100 will now be described. Measurement apparatus 100 performs measurement processing in a procedural order of reference gas measurement, base gas measurement, reference gas measurement, sample gas measurement, reference gas measurement, ... During measurement processing, auxiliary cell 11c is filled with reference gas and hermetically sealed.

In reference gas measurement, first sample cell 11a and second sample cell 11b are filled with reference gas by suction of reference gas into cylinder 21b and delivery of reference gas to cell 11 by gas injector 21, and infrared detectors 25a and 25b measure a quantity of light transmitted through ¹²CO₂ and ¹³CO₂, respectively. Since clean reference gas is fed to a gas flow path and cell 11 in reference gas measurement, the gas flow path and cell 11 can be cleaned. Since piston 21c is moved forward and rearward at this time, the inside of cylinder 21b can also be cleaned with clean reference gas.

In base gas measurement, first sample cell 11a and second sample cell 11b are filled with base gas by suction of base gas from breath bag B into cylinder 21b and delivery of base gas to cell 11 by gas injector 21, and infrared detectors 25a and 25b measure a quantity of light transmitted through ¹²CO₂ and ¹³CO₂, respectively.

In sample gas measurement, first sample cell 11a and second sample cell 11b are filled with sample gas by suction of sample gas from breath bag S into cylinder 21b and delivery of sample gas to cell 11 by gas injector 21, and infrared detectors 25a and 25b measure a quantity of light transmitted through ¹²CO₂ and ¹³CO₂, respectively.

As described previously, in measurement apparatus 100, signal processing circuit 40 processes signals provided from infrared detectors 25a and 25b and provides the signals to computing circuit 50. Therefore, measurement apparatus 100 finds the quantity of light transmitted through ¹²CO₂ and ¹³CO₂ that is measured in sample gas measurement, based on the signal processed by signal processing circuit 40.

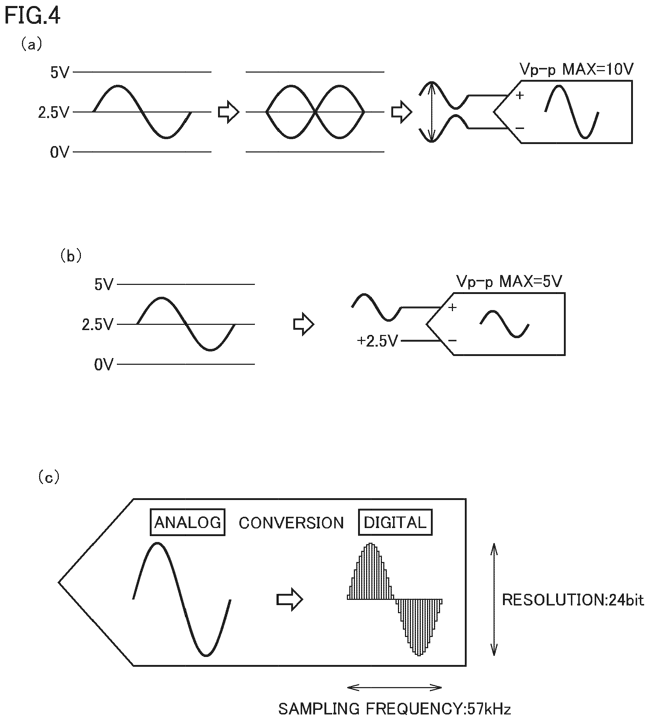

Signal processing in signal processing circuit 40 will now be described in detail. Fig. 4 is a diagram for illustrating signal processing in signal processing circuit 40 according to the present embodiment. Fig. 4 (a) shows signal processing for generating a differential signal in differential converter circuit 44 and Fig. 4 (b) shows signal processing for amplification by the amplifier circuit based on a reference signal, in comparison with Fig. 4 (a).

In Fig. 4 (a), when a sinusoidal signal that has passed through filter circuit 43 is provided to differential converter circuit 44, a signal reverse in phase (a reverse-phase signal) to the signal that has passed through filter circuit 43 (the passed signal) is generated. Differential converter circuit 44 provides the passed signal and the reverse-phase signal as a differential signal to AD conversion circuit 45. Since AD conversion circuit 45 receives input of the differential signal, the difference of the passed signal from the reverse-phase signal is a signal indicating a measurement result, and the AD conversion circuit converts the signal to a digital signal. Since a difference between the passed signal having a maximum amplitude from 0 V to 5 V and the reverse-phase signal having a maximum amplitude from 0 V to 5 V is taken as shown in Fig. 4 (a), AD conversion circuit 45 converts the signal having a maximum amplitude from 0 V to 10 V to the digital signal.

In Fig. 4 (b), a difference of the passed signal from a reference voltage is a signal indicating a measurement result and converted to a digital signal by AD conversion circuit 45. Since a difference between the passed signal having the maximum amplitude from 0 V to 5 V and the reference voltage at 2.5 V is taken, AD conversion circuit 45 converts a signal having the maximum amplitude of 5 V into a digital signal.

Therefore, when the difference of the passed signal from the reverse-phase signal is the signal indicating the measurement result in differential converter circuit 44 as shown in Fig. 4 (a), the signal indicating the measurement result is wider in dynamic range than in an example where the difference of the passed signal from the reference voltage is the signal indicating the measurement result as shown in Fig. 4 (b). Specifically, owing to differential converter circuit 44, the passed signal having the maximum amplitude from 0 V to 5 V becomes the signal having the maximum amplitude from 0 V to 10 V, which means the dynamic range wider by approximately two times.

When the difference of the passed signal from the reference voltage is the signal indicating the measurement result as shown in Fig. 4 (b), variation of the reference voltage due to an error directly affects the digital signal resulting from conversion in AD conversion circuit 45. On the other hand, when the difference of the passed signal from the reverse-phase signal is the signal indicating the measurement result in differential converter circuit 44 as shown in Fig. 4 (a), a difference between the reverse-phase signal and the passed signal is taken in spite of variation of the reference voltage due to an error and hence the error is canceled. Therefore, when differential converter circuit 44 generates a differential signal, the S/N ratio of the signal indicating the measurement result (the signal obtained by the detector) can be improved.

In Fig. 4 (c), when AD conversion circuit 45 converts a differential signal provided from differential converter circuit 44 to a digital signal, it converts the differential signal to the digital signal with a sampling frequency being set to 57 kHz and a resolution being set to 24 bits. In converting a signal processed by a conventional amplifier circuit to a digital signal, an AD conversion circuit converts the signal to the digital signal with the sampling frequency being set to 40 kHz and a resolution being set to 20 bits. Therefore, AD conversion circuit 45 can reduce a quantization error produced in AD conversion by converting the signal to the digital signal with the sampling frequency being set to 57 kHz and the resolution being set to 24 bits.

In particular, in converting a differential signal provided from differential converter circuit 44 to a digital signal, a dynamic range becomes wider. Therefore, the resolution should be made higher than in conversion of a passed signal to a digital signal. Specifically, when AD conversion circuit 45 converts a passed signal having the maximum amplitude from 0 V to 5 V to a digital signal at the resolution of 20 bits, in order to convert a signal having the maximum amplitude from 0 V to 10 V to a digital signal with a comparable quantization error, the passed signal should be converted to the digital signal at the resolution of at least 21 bits.

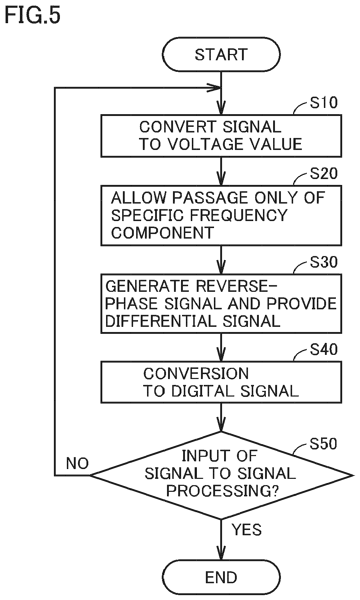

A signal processing method in signal processing circuit 40 will now be described with reference to a flowchart. Fig. 5 is a flowchart for illustrating a signal processing method in signal processing circuit 40 according to the present embodiment. Initially, signal processing circuit 40 converts in IV conversion circuit 41, a signal detected as a current value by infrared detector 25a or 25b to a signal of a voltage value (step S10). Infrared detectors 25a and 25b detect quantities of infrared light from light source apparatuses L1 and L2 with intensity thereof being sinusoidally modulated by optical chopper 22.

Then, signal processing circuit 40 has filter circuit 43 allow passage of a signal containing a specific frequency component among signals converted to voltage values in step S10 (step S20). The specific frequency component (a predetermined frequency component) refers to a frequency component (for example, 600 Hz) modulated by optical chopper 22.

Signal processing circuit 40 further generates in differential converter circuit 44, a reverse-phase signal of the passed signal that has passed through filter circuit 43 and provides a differential signal including the passed signal and the reverse-phase signal to AD conversion circuit 45 (step S30).

Then, signal processing circuit 40 converts in AD conversion circuit 45, the differential signal in step S30 to a digital signal (step S40). Thereafter, signal processing circuit 40 determines whether or not there is input of a signal to signal processing (step S50). When there is no input of a signal to signal processing (NO in step S50), signal processing circuit 40 quits signal processing. When there is input of a signal to signal processing (YES in step S50), the process returns to step S10.

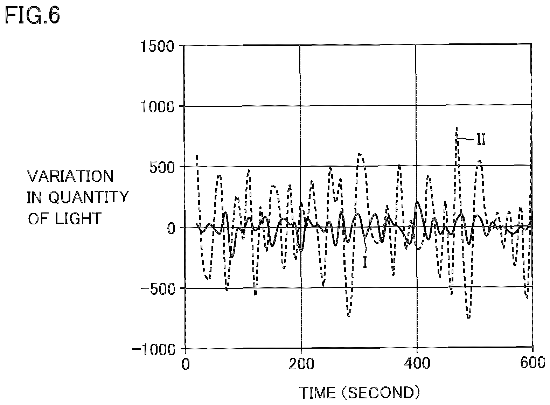

A result of improvement in S/N ratio of a signal indicating a measurement result (a signal obtained by the detector) by signal processing circuit 40 is shown. Fig. 6 shows a graph for illustrating an amount of variation of a signal processed by signal processing circuit 40 according to the present embodiment. With time being set on the abscissa and an amount of variation in quantity of light being set on the ordinate, the graph in Fig. 6 shows variation of the quantity of light measured by infrared detectors 25a and 25b and processed by signal processing circuit 40. The amount of variation in quantity of light is represented by an AD-converted count value.

In the graph shown in Fig. 6, a waveform I represents a measurement result obtained by emission of infrared light at constant intensity from light source apparatuses L1 and L2 and processing by signal processing circuit 40, of a signal based on the transmitted light quantity detected every ten seconds for 600 seconds by infrared detectors 25a and 25b. Waveform I represents an amount of variation of the measured quantity of light, and the amount of variation is substantially accommodated within a range from 0 "zero" to approximately 100.

A waveform II represents a measurement result obtained by emission of infrared light at constant intensity from light source apparatuses L1 and L2 and processing by a conventional amplifier circuit, of a signal based on the transmitted light quantity detected every ten seconds for 600 seconds by infrared detectors 25a and 25b. Waveform II represents an amount of variation of the measured quantity of light, and the amount of variation is varied within a range from 0 "zero" to approximately 500. Waveform II is larger in amount of variation than waveform I.

A standard deviation of variation in quantity of light represented by waveform I is 89, whereas a standard deviation of variation in quantity of light represented by waveform II is 387. Therefore, it can be seen that the standard deviation of variation in quantity of light represented by waveform I is at most approximately one third that of waveform II. Thus, when signal processing circuit 40 processes a signal based on the transmitted light quantity detected by infrared detectors 25a and 25b as shown with waveform I, the S/N ratio of the signal indicating the measurement result is improved and hence the amount of variation in quantity of light is reduced. In contrast, when the conventional amplifier circuit processes the signal based on the transmitted light quantity detected by infrared detectors 25a and 25b as shown with waveform II, the S/N ratio of the signal indicating the measurement result is poor and hence the amount of variation in quantity of light is large.

In measurement apparatus 100 according to the present embodiment, signal processing circuit 40 processes the signal based on the transmitted light quantity detected by infrared detectors 25a and 25b and the processed signal is handled as the measurement result. Therefore, the transmitted light quantity can be measured at a high S/N ratio in sample gas measurement. Therefore, with measurement apparatus 100, an amount of a sample necessary in measurement of gas can be reduced (for example, to half) and time necessary for measurement can be reduced.

Computing circuit 50 finds absorbance by CO₂ in base gas based on the quantity of light transmitted through reference gas measured in reference gas measurement and the quantity of light transmitted through base gas measured in base gas measurement. In reference gas measurement and base gas measurement, the quantity of light transmitted through each of ¹²CO₂ and ¹³CO₂ is measured. Therefore, computing circuit 50 can find the absorbance by each of ¹²CO₂ and ¹³CO₂ in base gas. Absorbance Abs by CO₂ in base gas can be calculated as Abs = -log[quantity of light transmitted through base gas/quantity of light transmitted through reference gas].

Similarly, computing circuit 50 finds absorbance by CO₂ in sample gas based on the quantity of light transmitted through reference gas measured in reference gas measurement and the quantity of light transmitted through sample gas measured in sample gas measurement. In reference gas measurement and sample gas measurement, the quantity of light transmitted through each of ¹²CO₂ and ¹³CO₂ is measured. Therefore, computing circuit 50 can find the absorbance by each of ¹²CO₂ and ¹³CO₂ in sample gas. Absorbance Abs by CO₂ in sample gas can be calculated as Abs = -log[quantity of light transmitted through sample gas/quantity of light transmitted through reference gas].

In finding the absorbance by CO₂, computing circuit 50 can cancel influence by drift during measurement by calculating an average value of the quantities of light transmitted through reference gas that are measured before and after base gas measurement or sample gas measurement.

Then, computing circuit 50 calculates a concentration of CO₂ based on the found absorbance by CO₂ by using a calibration curve. The calibration curve refers to a curve found with the least square method, by plotting results of measurement of the absorbance by CO₂ with respect to gas to be measured, a CO₂ concentration of which has been known. The calibration curve is prepared for each of ¹²CO₂ and ¹³CO₂. Therefore, computing circuit 50 uses the calibration curve for ¹²CO₂ to find the concentration of ¹²CO₂ in base gas based on the absorbance by ¹²CO₂ in base gas and to find the concentration of ¹²CO₂ in sample gas based on the absorbance by ¹²CO₂ in sample gas. Similarly, computing circuit 50 uses the calibration curve for ¹³CO₂ to find the concentration of ¹³CO₂ in base gas based on the absorbance by ¹³CO₂ in base gas and to find the concentration of ¹³CO₂ in sample gas based on the absorbance by ¹³CO₂ in sample gas.

Furthermore, computing circuit 50 calculates a concentration ratio between ¹²CO₂ and ¹³CO₂. Computing circuit 50 calculates the concentration ratio between ¹²CO₂ and ¹³CO₂ in base gas measurement and the concentration ratio between ¹²CO₂ and ¹³CO₂ in sample gas measurement.

In order to diagnose whether or not HP exists, computing circuit 50 calculates an amount of variation in concentration of ¹³CO₂ based on comparison between sample gas measurement and base gas measurement An amount of variation Δ¹³CO₂ in concentration of ¹³CO₂ is calculated as Δ¹³CO₂ = [(concentration ratio between ¹²CO₂ and ¹³CO₂ in sample gas measurement)-(concentration ratio between ¹²CO₂ and ¹³CO₂ in base gas measurement)] ×10³/[concentration ratio between ¹²CO₂ and ¹³CO₂ in base gas measurement] (unit: per mill (permillage)). When calculated amount of variation Δ¹³CO₂ in concentration of ¹³CO₂ is equal to or higher than 2.5 per mill, computing circuit 50 determines that HP is highly likely to exist in the stomach of the subject and makes determination as positive.

As set forth above, measurement apparatus 100 according to the present embodiment includes signal processing circuit 40 that detects with infrared detectors 25a and 25b, quantities of infrared light from light source apparatuses L1 and L2 sinusoidally modulated in intensity by optical chopper 22 and processes a signal based on the quantities of light detected by infrared detectors 25a and 25b. Signal processing circuit 40 includes IV conversion circuit 41 that converts a signal of a current value provided from infrared detectors 25a and 25b to a signal of a voltage value, filter circuit 43 that allows passage of a signal containing a frequency component modulated by optical chopper 22 among signals converted by IV conversion circuit 41, and differential converter circuit 44 that generates a reverse-phase signal of a passed signal that has passed through filter circuit 43 and provides a differential signal including the passed signal and the reverse-phase signal. Therefore, in measurement apparatus 100, signal processing circuit 40 can improve, by means of differential converter circuit 44 that provides a differential signal including the passed signal and the reverse-phase signal, the S/N ratio of the signal obtained by infrared detectors 25a and 25b, and a signal wider in dynamic range can be obtained.

Signal processing circuit 40 includes AD conversion circuit 45 that converts a differential signal provided from differential converter circuit 44 to a digital signal. With AD conversion circuit 45, signal processing circuit 40 can provide the digital signal readily processed by computing circuit 50 to computing circuit 50, and processing load imposed on computing circuit 50 can be mitigated. Naturally, without providing AD conversion circuit 45 in signal processing circuit 40, a differential signal provided from differential converter circuit 44 may directly be provided to computing circuit 50 and computing circuit 50 may convert the differential signal to a digital signal.

AD conversion circuit 45 sets a resolution at which the differential signal is converted to the digital signal to be higher than a resolution at which the passed signal is converted to the digital signal. AD conversion circuit 45 can thus convert the differential signal wider in dynamic range to the digital signal without increasing a quantization error. Naturally, AD conversion circuit 45 may convert the differential signal to the digital signal without setting the resolution to be higher.

Signal processing circuit 40 includes amplifier circuit 42 that amplifies a signal converted by IV conversion circuit 41 and provides a signal amplified by amplifier circuit 42 to filter circuit 43. With amplifier circuit 42, signal processing circuit 40 can amplify a signal provided from infrared detectors 25a and 25b to a signal that filter circuit 43 or differential converter circuit 44 can process. Naturally, so long as the signal converted by IV conversion circuit 41 is a signal that filter circuit 43 or differential converter circuit 44 can process, signal processing circuit 40 may provide a signal converted by IV conversion circuit 41 to filter circuit 43 without providing amplifier circuit 42.

The signal based on the quantity of light detected by infrared detectors 25a and 25b is a signal obtained by detection by infrared detectors 25a and 25b, of the quantity of infrared light from light source apparatuses L1 and L2 sinusoidally modulated in intensity by the light modulator. Though the light modulator is described as being optical chopper 22, the light modulator is not limited to optical chopper 22 so long as an apparatus can sinusoidally modulate intensity of infrared light from light source apparatuses L1 and L2.

Though infrared detectors 25a and 25b are described as each being a photovoltaic element, limitation to the photovoltaic element is not intended so long as a quantity of light can be detected, and a photoconductive element may be applicable.

### (Modification)

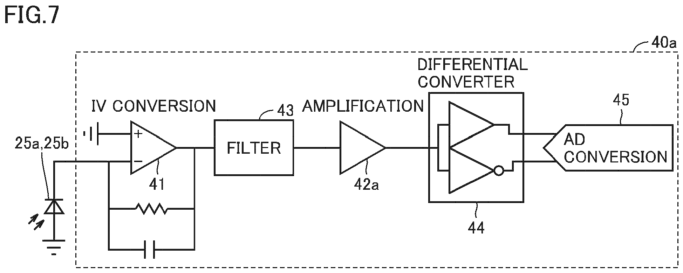

Signal processing circuit 40 shown in Fig. 2 is described as including amplifier circuit 42 that amplifies a signal converted by IV conversion circuit 41. Without being limited as such, signal processing circuit 40 may include an amplifier circuit at a position other than a position between IV conversion circuit 41 and filter circuit 43. Fig. 7 is a schematic diagram for illustrating a configuration of a signal processing circuit 40a according to a modification. Signal processing circuit 40a includes current-voltage conversion circuit (IV conversion circuit) 41, an amplifier circuit 42a, filter circuit 43, differential converter circuit (differential circuit) 44, and analog-digital conversion circuit (AD conversion circuit) 45. Components in signal processing circuit 40a the same as those in signal processing circuit 40 shown in Fig. 2 have the same reference characters allotted and detailed description will not be repeated.

Amplifier circuit 42a amplifies a signal that has passed through filter circuit 43 to a signal that differential converter circuit 44 can process. For example, when a signal that has passed through filter circuit 43 has a maximum amplitude from 0 V to 2.5 V, amplifier circuit 42a amplifies a voltage value thereof by approximately two times to obtain a signal having a maximum amplitude from 0 V to 5 V.

Signal processing circuit 40a includes amplifier circuit 42a that amplifies a signal, between filter circuit 43 and differential converter circuit 44 so as to amplify the signal that has passed through filter circuit 43 to a signal that differential converter circuit 44 can process.

Signal processing circuit 40a may be combined with amplifier circuit 42 provided in signal processing circuit 40 shown in Fig. 2. Specifically, signal processing circuit 40a may be provided with amplifier circuit 42 between IV conversion circuit 41 and filter circuit 43 and with amplifier circuit 42a that amplifies a signal between filter circuit 43 and differential converter circuit 44. A signal can thus be amplified to a signal necessary in each of filter circuit 43 and differential converter circuit 44.

Measurement apparatus 100 according to the present embodiment measures a component gas concentration ratio of gas to be measured that contains two types of component gas in isotopic relation with each other. Measurement apparatus 100 includes a cell (first sample cell 11a and second sample cell 11b), light source apparatuses L1 and L2, optical chopper 22, the optical filter (wavelength filters 24a and 24b), infrared detectors 25a and 25b, signal processing circuit 40 or 40a, and computing circuit 50.

Gas to be measured is stored in the cell (first sample cell 11a and second sample cell 1 lb). Light source apparatuses L1 and L2 emit infrared light to be transmitted through the cell. Optical chopper 22 sinusoidally modulates intensity of infrared light from light source apparatuses L1 and L2. The optical filter (wavelength filters 24a and 24b) allows transmission of a wavelength suitable for each type of component gas, of infrared light transmitted through the cell. Infrared detectors 24a and 25b detect a quantity of light transmitted through the optical filter. Signal processing circuit 40 or 40a processes the signal based on the quantity of light detected by infrared detector, 40a. Computing circuit 50 finds absorbance of a wavelength suitable for component gas based on the signal processed by signal processing circuit 40 or 40a and calculates the component gas concentration ratio.

An example in which measurement apparatus 100 according to the present embodiment with the configuration described previously measures a component gas concentration ratio of gas to be measured with carbon dioxide ¹³CO₂ and carbon dioxide ¹²CO₂ being defined as two types of component gas in isotopic relation with each other is described. In the configuration of measurement apparatus 100 described previously, however, any gas is applicable so long as a concentration ratio between two types of component gas in isotopic relation with each other can be measured.

An example in which signal processing circuit 40 or 40a is provided in measurement apparatus 100 that measures a component gas concentration ratio between two types of component gas, that is, carbon dioxide ¹³CO₂ and carbon dioxide ¹²CO₂, in isotopic relation with each other, is described. Signal processing circuit 40 or 40a can similarly be provided in an apparatus that requires detection by an infrared detector of a quantity of infrared light from a light source sinusoidally modulated in intensity by a light modulator and processing of a signal based on the quantity of light detected by the infrared detector.

It should be understood that the embodiment disclosed herein is illustrative and non-restrictive in every respect. The scope of the present invention is defined by the terms of the claims rather than the description above and is intended to include any modifications within the scope and meaning equivalent to the terms of the claims.

### REFERENCE SIGNS LIST

11 cell chamber; 11a first sample cell; 11b second sample cell; 11c auxiliary cell; 21 gas injector; 21a base; 21b cylinder; 21c piston; 21d nut; 21e feed screw; 21f pulse motor; 22 optical chopper; 22a motor; 24a, 24b wavelength filter; 25a, 25b infrared detector; 27 Peltier element; 28, 29 fan; 30 reference gas supply portion; 31 pressure gauge; 40, 40a signal processing circuit; 41 IV conversion circuit; 45 AD conversion circuit; 42, 42a amplifier circuit; 43 filter circuit; 44 differential converter circuit; 50 computing circuit; 100 measurement apparatus

## Claims

1. A signal processing circuit that processes a signal based on a quantity of light detected by an infrared detector, the signal processing circuit comprising:
a current-voltage conversion circuit that converts a signal of a current value provided from the infrared detector to a signal of a voltage value;
a filter circuit that allows passage of a signal containing a predetermined frequency component among signals converted by the current-voltage conversion circuit; and
a differential circuit that generates a reverse-phase signal of a passed signal that has passed through the filter circuit and provides a differential signal including the passed signal and the reverse-phase signal.

2. The signal processing circuit according to claim 1, further comprising an analog-digital conversion circuit that converts the differential signal provided from the differential circuit to a digital signal.

3. The signal processing circuit according to claim 2, wherein
the analog-digital conversion circuit sets a resolution at which the differential signal is converted to the digital signal to be higher than a resolution at which the passed signal is converted to the digital signal.

4. The signal processing circuit according to any one of claims 1 to 3, further comprising an amplifier circuit that amplifies a signal converted by the current-voltage conversion circuit, wherein
the signal amplified by the amplifier circuit is provided to the filter circuit.

5. The signal processing circuit according to any one of claims 1 to 3, further comprising an amplifier circuit that amplifies a signal, between the filter circuit and the differential circuit.

6. The signal processing circuit according to any one of claims 1 to 5, wherein
the signal based on the quantity of light detected by the infrared detector is a signal obtained by detection by the infrared detector, of a quantity of infrared light from a light source intensity of which is sinusoidally modulated by a light modulator.

7. The signal processing circuit according to any one of claims 1 to 6, wherein the infrared detector is a photovoltaic element.

8. A measurement apparatus that measures a component gas concentration ratio of gas to be measured that contains two types of component gas in isotopic relation with each other, the measurement apparatus comprising:
a cell in which the gas to be measured is stored;
a light source that emits infrared light to be transmitted through the cell;
a light modulator that sinusoidally modulates intensity of infrared light from the light source;
an optical filter that allows transmission of a wavelength suitable for each type of component gas, of infrared light transmitted through the cell;
an infrared detector that detects a quantity of light transmitted through the optical filter;
the signal processing circuit according to any one of claims 1 to 7 that processes a signal based on the quantity of light detected by the infrared detector; and
a computing circuit that finds absorbance of the wavelength suitable for the component gas based on the signal processed by the signal processing circuit and calculates the component gas concentration ratio.

9. The measurement apparatus according to claim 8, wherein
the two types of component gas contained in the gas to be measured are carbon dioxide ¹³CO₂ and carbon dioxide ¹²CO₂.

10. A signal processing method of processing a signal provided from an infrared detector, the signal processing method comprising:
converting a signal detected as a current value by the infrared detector to a signal of a voltage value;
allowing passage of a signal containing a predetermined frequency component among signals converted to voltage values; and
generating a reverse-phase signal of a signal that has passed through and providing a differential signal including the signal that has passed through and the reverse-phase signal to an analog-digital conversion circuit.
